# EUROPÄISCHE PATENTSCHRIFT

(11) **EP 4 523 629 B1**
(45) Veröffentlichungstag und Bekanntmachung des Hinweises auf die Patenterteilung: **29.10.2025**
(21) Anmeldenummer: 23197942.8
(22) Anmeldetag: 18.09.2023
(51) Int. Cl.: A61B 6/00

(54) **ABSCHÄTZUNG EINER STRAHLENBELASTUNGSKENNGRÖSSE FÜR EIN BILDGEBUNGSSYSTEM MIT EINER STRAHLUNGSQUELLE**
ESTIMATION OF A RADIATION EXPOSURE CHARACTERISTIC FOR AN IMAGING SYSTEM COMPRISING A RADIATION SOURCE
ESTIMATION D'UNE CARACTÉRISTIQUE DE CONTRAINTE DE FAISCEAU POUR UN SYSTÈME D'IMAGERIE COMPRENANT UNE SOURCE DE RAYONNEMENT

(43) Veröffentlichungstag der Anmeldung: 19.03.2025
(73) Patentinhaber: Siemens Healthineers AG, 91301 Forchheim (DE)
(72) Erfinder: Birkhold, Annette, 70193 Stuttgart (DE); Roser, Philipp, 91052 Erlangen (DE)
(74) Vertreter: Siemens Healthineers Patent Attorneys

(56) Entgegenhaltungen:
- CN-B- 104 545 957
- CN-B- 109 060 849
- US-A1- 2022 192 619

## Beschreibung

Die Erfindung betrifft ein Verfahren zur Abschätzung einer Strahlenbelastungskenngröße für ein Bildgebungssystem mit einer Strahlungsquelle, eine entsprechende Datenverarbeitungsvorrichtung, ein Bildgebungssystem mit einer Strahlungsquelle zum Erzeugen ionisierender Strahlung und einer solchen Datenverarbeitungsvorrichtung sowie ein entsprechendes Computerprogrammprodukt.

Bei Bildgebungssystemen mit einer Strahlungsquelle zum Erzeugen ionisierender Strahlung ist es beispielsweise aus regulatorischen Gründen wünschenswert, die Strahlenbelastung für Patienten und medizinisches Personal möglichst exakt zu dokumentieren. Dazu kann eine entsprechende Strahlenbelastungskenngröße im Betrieb des Bildgebungssystems gemessen werden.

Bei röntgenbasierten Bildgebungssystemen, etwa röntgenbasierten Angiographiesystemen kann dazu beispielsweise ein Dosisflächenprodukt, DAP (englisch: dose area product), als Strahlenbelastungskenngröße mittels einer als DAP-Kammer bezeichneten Messvorrichtung im Strahlengang des Bildgebungssystems gemessen werden. Das gemessene DAP kann neben den genannten regulatorischen Erfordernissen auch zur Regelung der Strahlungsquelle verwendet werden.

Solche DAP-Kammern oder entsprechende Messvorrichtungen zur Messung der Strahlenbelastungskenngröße führen, da sie im Strahlengang angeordnet sind, zu einer erhöhten Streuung der ionisierenden Strahlung. Dies kann zum einen die Strahlenbelastung des medizinischen Personals in der Umgebung des Bildgebungssystems erhöhen, und zum anderen die Bildqualität aufgrund zusätzlicher extrafokaler Strahlung reduzieren.

In der Veröffentlichung G. Poludniowski et al.: "Technical Note: SpekPy v2.0-a software toolkit for modeling x-ray tube spectra", Med. Phys. 48 (7), 3630, wird eine Software zur Modellierung von Röntgenröhrenspektren beschrieben und die Vorhersagen der Modellierung werden mit experimentell ermittelten Spektren abgeglichen.

Dokument CN 106291650 A beschreibt ein auf Monte-Carlo-Simulationen basierendes Verfahren zur Bestimmung einer Strahlentherapie-Dosis. Dazu wird ein virtuelles Quellenmodell zugrunde gelegt und basierend auf Ergebnissen eines Wassermodellexperiments korrigiert. Das korrigierte virtuelle Quellenmodell wird zum Berechnen der Strahlendosis in einem Tumorzielbereich verwendet.

Ferner ist eine Vielzahl von Möglichkeiten bekannt, aus patientenspezifischen Informationen, etwa Metadaten wie Körpergröße, Alter und so weiter, und/oder Bilddaten, wie etwa präinterventionellen CT-Rekonstruktionen und so weiter, statistische Patientenmodelle derart anzupassen oder zu erzeugen, dass ein patientenspezifisches Körpermodell für den individuellen Patienten resultiert. Solche Körpermodelle können je nach Komplexität Haut, Knochen, Gefäße, und/oder sonstige innere Organe beinhalten.

In der Veröffentlichung K. Shetty et al.: "PLIKS: A Pseudo-Linear Inverse Kinematic Solver for 3D Human Body Estimation" (arXiv:2211.11734v2) wird eine Methode zur Rekonstruktion eines 3D-Mesh des menschlichen Körpers aus einem einzigen 2D-Bild beschrieben. Die Methode basiert auf einer linearisierten Formulierung des parametrischen SMPL-Modells (englisch: skinned multi-person linear model). In der Veröffentlichung K. Shetty et. al.: "BOSS: Bones, Organs and Skin Shape Model" (arXiv:2303.04923v1) wird ein deformierbares menschliches Form- und Posenmodell vorgestellt, mit dem Haut, innere Organe und Knochen kombiniert und aus CT-Bildern erlernt werden können. Durch die Modellierung der statistischen Variationen in einem positionsnormalisierten Raum unter Verwendung der probabilistischen Hauptkomponentenanalyse und unter Beibehaltung der Gelenkkinematik wird eine ganzheitliche Darstellung des Körpers erreicht.

Außerdem sind im Stand der Technik Verfahren zur automatischen Einstellung der Röntgenbelichtung (Automatic Exposure Control) bekannt, z.B. aus der Druckschrift CN 104 545 957 B.

Es ist eine Aufgabe der vorliegenden Erfindung, die Streustrahlung im Betrieb eines Bildgebungssystems mit einer Strahlungsquelle zu reduzieren.

Diese Aufgabe wird durch den Gegenstand des unabhängigen Anspruchs gelöst. Weitere Ausführungsformen und vorteilhafte Weiterentwicklungen sind Gegenstand der abhängigen Ansprüche.

Die Erfindung beruht auf der Idee, eine Messvorrichtung zur Messung einer Strahlenbelastungskenngröße im regulären Betrieb des Bildgebungssystems durch eine modellbasierte **Ab**schätzung der Strahlenbelastungskenngröße zu ersetzen. Ausgehend von einem Basismodell zur Bestimmung der Strahlenbelastungskenngröße abhängig von einem Satz von Eingangsgrößen wird anhand von für das konkrete Bildgebungssystem gemessenen Werten der Strahlenbelastungskenngröße ein verfeinertes Modell erzeugt. Unter Verwendung des verfeinerten Modells wird die Strahlenbelastungskenngröße im regulären Betrieb abgeschätzt.

Gemäß einem Aspekt der Erfindung wird ein Verfahren zur Abschätzung einer Strahlenbelastungskenngröße für ein Bildgebungssystem mit einer Strahlungsquelle, insbesondere einer Strahlungsquelle zur Erzeugung ionisierender Strahlung, bereitgestellt. Dabei wird ein Basismodell zur Bestimmung der Strahlenbelastungskenngröße abhängig von einem Satz von Eingangsgrößen erhalten. Der Satz von Eingangsgrößen beinhaltet wenigstens einen Strahlungsquellenbetriebs-parameter. Es werden für das Bildgebungssystem gemessene Messdaten erhalten. Die Messdaten beinhalten die Strahlungsbelastungskenngröße für eine Vielzahl von unterschiedlichen Werten des wenigstens einen Strahlungsquellenbetriebsparameters. Es wird ein verfeinertes Modell erzeugt, insbesondere ein verfeinertes Modell zur Bestimmung der Strahlenbelastungskenngröße abhängig von dem Satz von Eingangsgrößen, indem das Basismodell abhängig von den Messdaten angepasst wird. Es werden aktuelle Werte für den Satz von Eingangsgrößen für das Bildgebungssystem bestimmt. Mittels des verfeinerten Modells wird abhängig von den aktuellen Werten für den Satz von Eingangsgrößen ein Schätzwert für die Strahlenbelastungskenngröße bestimmt.

Die eigentliche Durchführung der Messungen zum Bestimmen der der Messdaten ist nicht notwendigerweise Teil des erfindungsgemäßen Verfahrens. Die Messdaten können im Rahmen des erfindungsgemäßen Verfahrens beispielsweise gespeichert auf einem Datenträger erhalten werden. Dementsprechend kann das erfindungsgemäße Verfahren als rein computerimplementiertes Verfahren ausgestaltet sein. Sofern nicht anders angegeben, können alle Schritte des computerimplementierten Verfahrens von einer Datenverarbeitungsvorrichtung durchgeführt werden, welche wenigstens eine Recheneinheit aufweist. Insbesondere ist die wenigstens eine Recheneinheit zur Durchführung der Schritte des computerimplementierten Verfahrens konfiguriert oder angepasst. Hierzu kann die wenigstens eine Recheneinheit beispielsweise eines oder mehrere Computerprogramme speichern, deren Ausführung die wenigstens eine Recheneinheit dazu veranlassen, das computerimplementierte Verfahren durchzuführen.

In anderen Ausführungsformen des erfindungsgemäßen Verfahrens beinhaltet dieses das Bestimmen der Messdaten, also das Messen der Strahlenbelastungskenngröße für das Bildgebungssystem für die Vielzahl von verschiedenen Werten des wenigstens einen Strahlungsquellenbetriebsparameters. Dementsprechend ergibt sich aus jeder rein computerimplementierten Ausgestaltungsform des erfindungsgemäßen Verfahrens direkt ein entsprechendes Verfahren zur Abschätzung einer Strahlenbelastungskenngröße, das nicht rein computerimplementiert ist beziehungsweise umgekehrt.

In beiden Fällen handelt es sich bei den Messdaten jedoch um für das konkrete individuelle Bildgebungssystem beziehungsweise dessen Strahlungsquelle gemessene Werte für die Strahlungsbelastungskenngröße. Demzufolge kann das verfeinerte Modell als stückspezifisches Modell für das konkrete individuelle Bildgebungssystem aufgefasst werden. Dies ist im Allgemeinen für das Basismodell so nicht der Fall. Das Basismodell kann beispielsweise Bildgebungssysteme einer bestimmten Art oder mit einer bestimmten Art von Strahlungsquelle modellieren. Es ist auch möglich, dass das Basismodell einen bestimmten Modelltyp des Bildgebungssystems modelliert oder eine bestimmte Produktionsserie. Beispielsweise kann das Basismodell während einer Entwicklungsphase, insbesondere zur Entwicklung des Bildgebungssystems, erstellt werden. Das verfeinerte Modell kann beispielsweise während oder am Ende einer Produktion des konkreten Bildgebungssystems erzeugt werden.

Das Basismodell kann als Eingangsdaten die entsprechenden Werte eines Satzes der Eingangsgrößen erhalten und basierend darauf beziehungsweise abhängig davon die Strahlenbelastungskenngröße berechnen. Das Basismodell kann also als Funktion oder Abbildung von dem Satz von Eingangsgrößen auf die Strahlenbelastungskenngröße verstanden werden. Dies gilt analog auch für das verfeinerte Modell.

Die Strahlenbelastungskenngröße ist ein Maß für die Strahlenbelastung, der ein Objekt oder eine Person außerhalb der Strahlungsquelle ausgesetzt ist, insbesondere an einer definierten Position oder in einem definierten Bereich. Die Strahlungsbelastungskenngröße betrifft insbesondere eine Dosis oder Energie der ionisierenden Strahlung in einem definierten Messbereich außerhalb der Strahlungsquelle. Die Strahlenbelastungskenngröße kann beispielsweise einer Energie pro Flächeneinheit und/oder pro Masseneinheit und so weiter entsprechen. Die Strahlenbelastungskenngröße kann beispielsweise einer Kerma entsprechen oder einem Dosisflächenprodukt, DAP.

Zum Erzeugen der Messdaten werden die Werte des wenigstens einen Strahlungsquellenbetriebsparameters variiert und die Strahlenbelastungskenngröße wird entsprechen gemessen. Neben der Variation des wenigstens einen Strahlungsquellenbetriebsparameters können auch sonstige Parameter oder Größen des Satzes von Eingangsgrößen, insbesondere alle Größen des Satzes von Eingangsgrößen, variiert werden, um die Strahlenbelastungskenngröße dementsprechend jeweils zu messen.

Der wenigstens eine Strahlungsquellenbetriebsparameter entspricht insbesondere wenigstens einem Parameter, der für die Strahlungsquelle eingestellt wird oder für die Strahlungsquelle gegeben ist, um die ionisierende Strahlung zu erzeugen.

Handelt es sich bei der Strahlungsquelle beispielsweise um eine Röntgenstrahlungsquelle, also insbesondere eine Röntgenröhre, so kann der wenigstens eine Strahlungsquellenbetriebsparameter insbesondere eine Spitzenkilovoltspannung, kVp (englisch: peak kilovoltage), also eine maximale Röhrenspannung, die beim Erzeugen der Röntgenstrahlung als ionisierende Strahlung an die Röntgenröhre angelegt wird, beinhalten. Der wenigstens eine Strahlungsquellenbetriebsparameter kann auch einen Röhrenstrom der Röntgenröhre und/oder eine Brennfleckgröße (englisch: focal spot size) und so weiter beinhalten.

Dass das verfeinerte Modell erzeugt wird, indem das Basismodell abhängig von den Messdaten angepasst wird, kann beispielsweise auch derart verstanden werden, dass das verfeinerte Modell basierend auf den Messdaten unter Berücksichtigung des Basismodells, insbesondere unter Berücksichtigung des Basismodells als Startpunkt oder Startzustand, erzeugt wird. Das Basismodell kann beispielsweise durch eine Vielzahl von Modellparametern festgelegt sein und die Anpassung des Basismodells abhängig von den Messdaten kann derart erfolgen, dass die Modellparameter derart angepasst werden, dass das verfeinerte Modell die Messdaten korrekt reproduzieren kann beziehungsweise der Fehler bei der Reproduktion der Messdaten im Vergleich zum Basismodell reduziert wird. Die Modellparameter können auch Gewichtungsfaktoren oder Bias-Faktoren eines neuronalen Netzwerks sein, falls das Basismodell ein solches neuronales Netzwerk beinhaltet. Es ist jedoch nicht notwendig, dass das Basismodell auf einem neuronalen Netzwerk oder einem anderen maschinell trainierbaren Algorithmus basiert. Die Modellparameter können dementsprechend auch Polynomkoeffizienten eines Polynommodells oder andere Kurvenparameter der oben genannten Funktion oder Abbildung sein.

Unter Verwendung des verfeinerten Modells kann im regulären Betrieb, beispielsweise im wissenschaftlichen oder klinischen Betrieb, des Bildgebungssystems weitgehend auf Hardware, wie zum Beispiel eine DAP-Kammer, im Strahlengang zum Bestimmen der Strahlungsbelastungskenngröße verzichtet werden. Die Menge an gesteuerter ionisierender Strahlung wird somit reduziert.

Wie eingangs erwähnt, kann die Strahlenbelastungskenngröße zur Dokumentation der Strahlenbelastung von Patienten oder medizinischem Personal verwendet werden. Es ist jedoch beispielsweise auch möglich, die Strahlenbelastungskenngröße zur Regelung der Strahlungsquelle zu verwenden oder für sonstige Zwecke.

Da das verfeinerte Modell basierend auf den Messdaten für das konkrete Bildgebungssystem erzeugt wird, kann die Abschätzung der Strahlenbelastungskenngröße insbesondere auch gegenüber einer Abschätzung erhöht werden, die eine ideale Strahlungsquelle oder ein idealisiertes Bildgebungssystem basierend ausschließlich auf physikalischen Modellen basiert. Gegenüber hochgenauen physikalischen Simulationen zur Berechnung der Strahlenbelastungskenngröße, beispielsweise unter Verwendung von Monte-Carlo-Verfahren, können durch das erfindungsgemäße Verfahren Rechenressourcen, insbesondere Rechenzeit, in erheblichem Maße eingespart werden.

Gemäß der Erfindung werden nach Ablauf eines Aktualisierungszeitraums nach einer Messung der Messdaten für das Bildgebungssystem gemessene weitere Messdaten erhalten, beispielsweise gemessen. Die weiteren Messdaten beinhalten die Strahlungsbelastungskenngröße für eine weitere Vielzahl von verschiedenen Werten des wenigstens einen Strahlungsquellenbetriebsparameters. Es wird ein aktualisiertes Modell erzeugt, indem das verfeinerte Modell abhängig von den weiteren Messdaten angepasst wird. Weitere Werte für den Satz von Eingangsgrößen für das Bildgebungssystem werden bestimmt und mittels des aktualisierten Modells wird abhängig von den weiteren Werten für den Satz von Eingangsgrößen ein weiterer Schätzwert für die Strahlenbelastungskenngröße bestimmt.

Ob und wann der Aktualisierungszeitraum abgelaufen ist, kann im Allgemeinen basierend auf vorgegebenen Zeitintervallen und/oder basierend auf Überwachungsmessungen an dem Bildgebungssystem entschieden werden.

Die Erläuterungen zu den Messdaten können analog auf die weiteren Messdaten übertragen werden. Das aktualisierte Modell kann insbesondere basierend auf dem verfeinerten Modell in analoger Weise erzeugt werden, wie das verfeinerte Modell zuvor basierend auf dem Basismodell erzeugt wurde. Entsprechende Erläuterungen können analog übertragen werden.

Die Strahlenbelastungskenngröße hängt von den Werten des Satzes von Eingangsgrößen und insbesondere den Werten des wenigstens einen Strahlungsquellenbetriebsparameters ab. Diese Abhängigkeit kann sich jedoch aufgrund von Alterungseffekten, insbesondere der Strahlungsquelle, beispielsweise der Anode im Falle einer Röntgenröhre, und/oder sonstiger Komponenten des Bildgebungssystems, beispielsweise zur Filterung der ionisierenden Strahlung und/oder Strahlformung und so weiter, verändern. Durch die Erzeugung des aktualisierten Modells in entsprechenden Ausführungsformen des erfindungsgemäßen Verfahrens können daher Abweichungen und Ungenauigkeiten des verfeinerten Modells, die sich aufgrund der genannten Alterungseffekte im Laufe der Zeit einstellen können, wenigstens zum Teil kompensiert werden.

Gemäß zumindest einer Ausführungsform werden für wenigstens ein Referenzbildgebungssystem gemessene Referenzmessdaten erhalten. Die Referenzmessdaten beinhalten die Strahlungsbelastungskenngröße für eine Vielzahl von verschiedenen Werten des wenigstens einen Strahlungsquellenbetriebsparameters. Das Basismodell wird basierend auf den Referenzmessdaten erzeugt.

Die Erläuterungen zu den Messdaten können analog auf die Referenzmessdaten übertragen werden, wobei die Referenzmessdaten aus Messungen für mehrere Referenzbildgebungssysteme stammen können und/oder wiederholt zu unterschiedlichen Zeitpunkten erfasst werden können, beispielsweise um Alterungseffekte und/oder Temperatureinflüsse zu berücksichtigen.

Die Referenzbildgebungssysteme können vom gleichen Typ oder gleichen Modell oder einem ähnlichen oder vergleichbaren Modell sein wie das Bildgebungssystem. Beispielsweise kann die Art der Strahlungsquelle, beispielsweise eine Röntgenquelle, für das Referenzbildgebungssystem und das Bildgebungssystem gleich sein. Die einzelnen Referenzbildgebungssysteme müssen jedoch nicht notwendigerweise identisch zum Bildgebungssystem sein. Das Basismodell ist also ein allgemeineres verglichen mit dem verfeinerten Modell, welches konkret für das individuelle Bildgebungssystem angepasst ist. Dementsprechend kann das Basismodell für verschiedene Bildgebungssysteme verwendet werden, um entsprechende verschiedene stückspezifische verfeinerte Modelle zu erzeugen wie oben beschrieben.

Gemäß zumindest einer Ausführungsform wird ein physikalisches Initialmodell zur Bestimmung der Strahlenbelastungskenngröße abhängig von dem Satz von Eingangsgrößen erhalten. Das Basismodell wird erzeugt, indem das physikalische Initialmodell abhängig von den Referenzmessdaten angepasst wird.

Bei dem physikalischen Initialmodell handelt es sich insbesondere um ein basierend auf physikalischen Zusammenhängen, insbesondere physikalischen Zusammenhängen bei der Erzeugung der ionisierenden Strahlung mittels der Strahlungsquelle, erzeugtes Modell. Das physikalische Initialmodell kann dabei auch Näherungen beinhalten. Insbesondere handelt es sich bei dem physikalischen Initialmodell nicht um einen vollständig oder im Wesentlichen durch maschinelles Lernen trainierten Algorithmus.

Das physikalische Initialmodell kann beispielsweise durch einen Satz von Modellparametern definiert oder festgelegt sein. Das Erzeugen des Basismodells erfolgt dann insbesondere durch Anpassung des Satzes von Modellparametern gemäß den Referenzmessdaten. Analog kann das verfeinerte Modell durch erneute Anpassung des Satzes von Modellparametern entsprechend der Messdaten erzeugt werden.

Gemäß zumindest einer Ausführungsform wird das Basismodell erzeugt, indem ein durch maschinelles Lernen trainierbarer Algorithmus basierend auf den Referenzmessdaten trainiert wird. Insbesondere ist das Basismodell der durch das maschinelle Lernen trainierte Algorithmus.

Der trainierbare Algorithmus kann beispielsweise eines oder mehrere künstliche neuronale Netzwerke und/oder eine oder mehrere Support Vector Maschinen, SVM, beinhalten.

Das Trainieren des trainierbaren Algorithmus basierend auf den Referenzmessdaten kann gemäß einem bekannten Trainingsverfahren beispielsweise für neuronale Netzwerke oder SVMs erfolgen.

In solchen Ausführungsformen kann auch das verfeinerte Modell erzeugt werden, indem der basierend auf den Referenzmessdaten trainierte Algorithmus basierend auf den Messdaten erneut oder weitergehend trainiert wird. Es ist jedoch auch möglich, dass das Basismodell durch den trainierten Algorithmus gegeben ist und das verfeinerte Modell dennoch basierend auf physikalischen Modellen erzeugt wird oder umgekehrt.

Da trainierbare Algorithmen, insbesondere künstliche neuronale Netzwerke, als universelle Näherungen für Funktionen aufgefasst werden können, kann das Basismodell in solchen Ausführungsformen unabhängig davon mit hoher Genauigkeit erzeugt werden, ob die physikalischen Zusammenhänge zur Erzeugung der ionisierenden Strahlung und insbesondere der Einfluss von Alterungseffekten oder dergleichen bekannt ist oder nicht.

Gemäß zumindest einer Ausführungsform beinhaltet der Satz von Eingangsgrößen wenigstens einen Zustands- und/oder Materialparameter für eine Strahlungsfiltervorrichtung des Bildgebungssystems. Die Messdaten beinhalten die Strahlungsbelastungskenngröße für eine Vielzahl von verschiedenen Werten des wenigstens einen Zustands und/oder Materialparameters.

In verschiedenen Ausführungsformen gilt dies analog für die Referenzmessdaten und/oder die weiteren Messdaten.

Die Strahlungsfiltervorrichtung kann beispielsweise einen oder mehrere Filter zur Einbringung in den Strahlengang, insbesondere zwischen einem Austrittsbereich der ionisierenden Strahlung aus der Strahlungsquelle und einem zu untersuchenden beziehungsweise abzubildenden Objekt, handeln. Im Falle eines röntgenbasierten Bildgebungssystems und einer Röntgenröhre als Strahlungsquelle kann der wenigstens eine Filter beispielsweise einen oder mehrere homogene Metallfilter zur Strahlaufhärtung, also zum Herausfiltern niedrigenergetischer Anteile, beinhalten und/oder einen oder mehrere Keilfilter und so weiter.

Der wenigstens eine Zustands- und/oder Materialparameter für die Strahlungsfiltervorrichtung kann beispielsweise eine Filterschichtdicke, ein Material des jeweiligen Filters, eine Position des Filters, insbesondere im Falle eines Keilfilters, und so weiter handeln.

Dementsprechend kann das verfeinerte Modell auch Alterungseffekten der Strahlungsfiltervorrichtung, beispielsweise durch Materialveränderungen des Filtermaterials aufgrund der Röntgenstrahlung und so weiter, genau berücksichtigt werden.

Gemäß zumindest einer Ausführungsform beinhaltet der Satz von Eingangsgrößen wenigstens einen Zustands- und/oder Materialparameter für eine Strahlformungsvorrichtung des Bildgebungssystems. Die Messdaten beinhalten die Strahlungsbelastungskenngröße für eine Vielzahl von verschiedenen Werten des wenigstens einen Zustands und/oder Materialparameters.

In verschiedenen Ausführungsformen gilt dies analog für die Referenzmessdaten und/oder die weiteren Messdaten.

Die Strahlformungsvorrichtung kann beispielsweise einen oder mehrere Kollimatoren zur Einbringung in den Strahlengang, insbesondere zwischen der Strahlungsfiltervorrichtung und dem Objekt, handeln.

Der wenigstens eine Zustands- und/oder Materialparameter für die Strahlformungsvorrichtung kann beispielsweise eine Dicke oder Geometrie eines Kollimators, ein Material des jeweiligen Kollimators, eine Öffnungsgröße des Kollimators und so weiter handeln.

Dementsprechend kann das verfeinerte Modell auch Alterungseffekten der Strahlformungsvorrichtung, beispielsweise durch Materialveränderungen eines Kollimators aufgrund der Röntgenstrahlung und so weiter, genau berücksichtigt werden.

Gemäß zumindest einer Ausführungsform beinhaltet der Satz von Eingangsgrößen eine Umgebungstemperatur der Strahlungsquelle und die Messdaten beinhalten die Strahlungsbelastungskenngröße für eine Vielzahl verschiedener Werte für die Umgebungstemperatur.

Auf diese Weise können auch temperaturabhängige Effekte, die einen Einfluss auf die Strahlenbelastungskenngröße haben können, berücksichtigt werden. Die Umgebungstemperatur kann dabei insbesondere einer Lufttemperatur eines Raums, in dem sich die Strahlungsquelle befindet, oder einer Lufttemperatur in einem Gehäuse, in dem sich die Strahlungsquelle befindet, entsprechen.

Gemäß zumindest einer Ausführungsform beinhaltet der Satz von Eingangsgrößen eine Temperatur wenigstens eines Bauteils, insbesondere des Bildgebungssystems, entsprechen, das sich beispielsweise in der Umgebung der Strahlungsquelle befindet, und die Messdaten beinhalten die Strahlungsbelastungskenngröße für eine Vielzahl verschiedener Werte für die Temperatur des wenigstens einen Bauteils.

Auf diese Weise können auch temperaturabhängige Effekte, die einen Einfluss auf die Strahlenbelastungskenngröße haben können, berücksichtigt werden. Insbesondere kann das wenigstens eine Bauteil dabei durch Wärmeabgabe den Wert der Strahlenbelastungskenngröße beeinflussen.

Gemäß zumindest einer Ausführungsform beinhaltet der Satz von Eingangsgrößen wenigstens einen Belichtungsparameter und die Messdaten beinhalten die Strahlenbelastungskenngröße für eine Vielzahl von verschiedenen Werten des wenigstens einen Belichtungsparameters.

Dies gilt gegebenenfalls analog für die Referenzmessdaten und/oder die weiteren Messdaten.

Die Belichtungsparameter werden hier und im Folgenden als solche bezeichnet, obwohl es sich bei der ionisierenden Strahlung im Allgemeinen nicht um Licht im eigentlichen Sinne handeln muss, sondern es sich beispielsweise auch um Röntgenstrahlung handeln kann. Das Verständnis des Begriffs der Belichtung lässt sich allerdings analog auch auf andere elektromagnetische Strahlung, wie beispielsweise Röntgenstrahlung, übertragen. Der wenigstens eine Belichtungsparameter kann beispielsweise eine Belichtungsdauer oder eine Blendenöffnung bei der Belichtung beinhalten oder eine Pulsbreite eines Pulses der ionisierenden Strahlung bei der Belichtung und so weiter.

Auf diese Weise kann das verfeinerte Modell für unterschiedliche Einstellungen bei der Belichtung genau erzeugt werden.

Gemäß zumindest einer Ausführungsform ist die Strahlenbelastungskenngröße ein Dosisflächenprodukt oder eine Kerma oder eine von dem Dosisflächenprodukt und/oder der Kerma abhängige Größe.

Beispielsweise handelt es sich bei der Strahlungsquelle in diesem Fall um eine Röntgenröhre. Analoge Größen ergeben sich jedoch auch für andere ionisierende Strahlungstypen.

Gemäß der Erfindung werden mittels des Bildgebungssystems erzeugte Bilddaten, welche ein abzubildendes Objekt, insbesondere einen Körper eines Patienten, darstellen, erhalten. Es wird außerdem ein Körpermodell für das Objekt, insbesondere für den Körper des Patienten, erhalten. Abhängig von den Bilddaten, abhängig von dem Körpermodell, abhängig von für die Erzeugung der Bilddaten verwendeten Werten für den Satz von Eingangsgrößen und abhängig von dem verfeinerten Modell wird eine Konsistenzprüfung durchgeführt. Abhängig von einem Ergebnis der Konsistenzprüfung wird festgestellt, ob der Aktualisierungszeitraum abgelaufen ist.

Insbesondere wird also abhängig von dem Ergebnis der Konsistenzprüfung ein Konsistenzmaß bestimmt und abhängig von dem Konsistenzmaß wird entschieden, ob der Aktualisierungszeitraum abgelaufen ist oder nicht.

Der Aktualisierungszeitraum ist also kein fest vorgegebenes Zeitintervall, sondern wird gemäß der Erfindung abhängig von den konkreten gegenwärtigen Eigenschaften des Bildgebungssystems, insbesondere den Bilddaten, bestimmt.

Die Konsistenzprüfung beinhaltet insbesondere eine Überprüfung dahingehend, ob die Bilddaten, das Körpermodell, die zur Erzeugung der Bilddaten verwendeten Werte für den Satz von Eingangsgrößen und das verfeinerte Modell untereinander konsistent sind.

Wird beispielsweise festgestellt, dass diese konsistent sind und dementsprechend festgestellt, dass der Aktualisierungszeitraum nicht abgelaufen ist, kann das verfeinerte Modell beispielsweise weiterhin beibehalten werden. Andererseits, falls festgestellt wird, dass die Konsistenz nicht oder nicht ausreichend gegeben ist, der Aktualisierungszeitraum also dementsprechend abgelaufen ist, kann das aktualisierte Modell abhängig von dem verfeinerten Modell erzeugt werden, wie oben beschrieben.

Dass das Körpermodell erhalten wird, kann insbesondere derart verstanden werden, dass es in computerlesbarer Form bereitgestellt, insbesondere gespeichert, wird. Das erfindungsgemäße Verfahren kann aber auch das Erzeugen oder teilweise Erzeugen des Körpermodells, beispielsweise durch Anpassung eines vorgegebenen statistischen Körpermodells, beinhalten.

Das Körpermodell entspricht beispielsweise einer dreidimensionalen räumlich aufgelösten, beispielsweise in Form eines Voxelmodells gegebenen, Verteilung eines Abschwächungskoeffizienten für die jeweilige ionisierende Strahlung.

Die Konsistenzprüfung kann in verschiedener Weise durchgeführt werden. Gemäß zumindest einer Ausführungsform werden abhängig von dem Körpermodell, abhängig von den für die Erzeugung der Bilddaten verwendeten Werten für den Satz von Eingangsgrößen und abhängig von dem verfeinerten Modell simulierte Bilddaten erzeugt und zur Konsistenzprüfung werden die simulierten Bilddaten mit den Bilddaten abgeglichen.

Das Ergebnis der Konsistenzprüfung beinhaltet also insbesondere eine Abweichung der Bilddaten von den simulierten Bilddaten. Ist die Abweichung größer als ein vorgegebener Grenzwert, so kann entschieden werden, dass der Aktualisierungszeitraum abgelaufen ist.

Gemäß zumindest einer Ausführungsform wird abhängig von den Bilddaten, abhängig von dem Körpermodell und abhängig von den für die Erzeugung der Bilddaten verwendeten Werten für den Satz von Eingangsgrößen ein Vergleichsmodell erzeugt und zur Konsistenzprüfung wird das Vergleichsmodell mit dem verfeinerten Modell abgeglichen.

Das Ergebnis der Konsistenzprüfung beinhaltet also insbesondere eine Abweichung des Vergleichsmodells von dem verfeinerten Modell, beispielsweise eine Abweichung der entsprechenden Modellparameter. Ist diese Abweichung größer als ein vorgegebener Grenzwert, so kann entschieden werden, dass der Aktualisierungszeitraum abgelaufen ist.

Gemäß zumindest einer Ausführungsform wird abhängig von den Bilddaten, abhängig von den für die Erzeugung der Bilddaten verwendeten Werten für den Satz von Eingangsgrößen und abhängig von dem verfeinerten Modell ein Vergleichskörper-modell erzeugt und zur Konsistenzprüfung wird das Vergleichskörpermodell mit dem Körpermodell abgeglichen.

Das Ergebnis der Konsistenzprüfung beinhaltet also insbesondere eine Abweichung des Vergleichskörpermodells von dem Körpermodell. Ist diese Abweichung größer als ein vorgegebener Grenzwert, so kann entschieden werden, dass der Aktualisierungszeitraum abgelaufen ist.

Gemäß zumindest einer Ausführungsform beinhaltet die Konsistenzprüfung eine Anwendung eines weiteren durch maschinelles Lernen trainierten Algorithmus auf von den Bilddaten, dem Körpermodell, den für die Erzeugung der Bilddaten verwendeten Werten für den Satz von Eingangsgrößen und dem verfeinerten Modell abhängige Eingabedaten.

Eine Ausgabe des weiteren trainierten Algorithmus entspricht dann dem Ergebnis der Konsistenzprüfung, also einem Maß für die Konsistenz der Eingabedaten.

Gemäß zumindest einer Ausführungsform ist das Bildgebungssystem ein röntgenbasiertes Bildgebungssystem und die Strahlungsquelle ist eine Röntgenstrahlungsquelle, insbesondere eine Röntgenröhre.

Gemäß einem weiteren Aspekt der Erfindung wird eine Datenverarbeitungsvorrichtung angegeben. Die Datenverarbeitungsvorrichtung weist wenigstens eine Recheneinheit auf, die dazu eingerichtet ist, ein erfindungsgemäßes Verfahren, insbesondere ein computerimplementiertes erfindungsgemäßes Verfahren, durchzuführen.

Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Gemäß einem weiteren Aspekt der Erfindung wird ein Bildgebungssystem angegeben. Das Bildgebungssystem weist eine erfindungsgemäße Datenverarbeitungsvorrichtung, eine Strahlungsquelle zum Erzeugen ionisierender Strahlung, sowie einen Strahlungsdetektor zum Detektieren von Teilen der ionisierenden Strahlung, insbesondere von durch das abzubildende Objekt hindurchgetretenen Teilen der ionisierenden Strahlung.

Weitere Ausführungsformen des erfindungsgemäßen Bildgebungssystems folgen unmittelbar aus den verschiedenen Ausgestaltungen des erfindungsgemäßen Verfahrens und umgekehrt. Insbesondere lassen sich einzelne Merkmale und entsprechende Erläuterungen sowie Vorteile bezüglich der verschiedenen Ausführungsformen zu dem erfindungsgemäßen Verfahren analog auf entsprechende Ausführungsformen des erfindungsgemäßen Bildgebungssystems übertragen.

Gemäß einem weiteren Aspekt der Erfindung wird ein Computerprogramm mit Befehlen angegeben. Wenn die Befehle durch wenigstens Datenverarbeitungsvorrichtung, insbesondere eine **er**findungsgemäße Datenverarbeitungsvorrichtung, ausgeführt werden, veranlassen die Befehle die Datenverarbeitungsvorrichtung dazu, ein erfindungsgemäßes Verfahren durchzuführen.

Die Befehle können beispielsweise als Programmcode vorliegen. Der Programmcode kann beispielsweise als Binärcode oder Assembler und/oder als Quellcode einer Programmiersprache, zum Beispiel **C,** und/oder als Programmskript, zum Beispiel Python, bereitgestellt sein.

Gemäß einem weiteren Aspekt der Erfindung wird ein computerlesbares Speichermedium angegeben, das ein erfindungsgemäßes Computerprogramm speichert.

Das Computerprogramm und das computerlesbare Speichermedium sind jeweils Computerprogrammprodukte mit den Befehlen.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen:
- FIG 1: eine schematische Darstellung einer beispielhaften Ausführungsform eines erfindungsgemäßen Bildgebungssystems;
- FIG 2: ein schematisches Ablaufdiagramm einer beispielhaften Ausführungsform eines erfindungsgemäßen Verfahrens zur Abschätzung einer Strahlenbelastungskenngröße;
- FIG 3: ein schematisches Ablaufdiagramm einer weiteren beispielhaften Ausführungsform eines erfindungsgemäßen Verfahrens zur Abschätzung einer Strahlenbelastungskenngröße; und
- FIG 4: eine schematische Darstellung der Messung einer Strahlenbelastungskenngröße gemäß einer weiteren beispielhaften Ausführungsform eines erfindungsgemäßen Verfahrens zur Abschätzung einer Strahlenbelastungskenngröße.

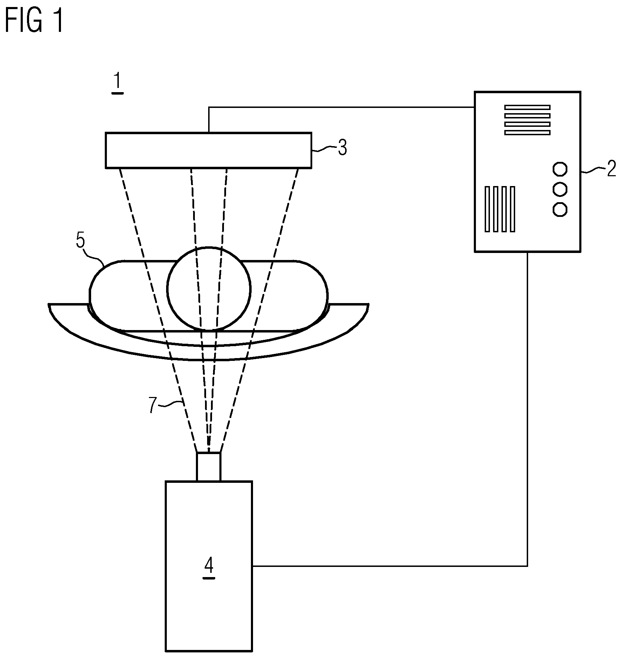

In FIG 1 ist schematisch eine beispielhafte Ausführungsform eines erfindungsgemäßen Bildgebungssystems 1 dargestellt. Das Bildgebungssystem 1 weist wenigstens eine Recheneinheit 2 auf, sowie eine Bildgebungsmodalität, die eine Strahlungsquelle 4 zum Erzeugen ionisierender Strahlung 7 und einen Strahlungsdetektor 3 zum Detektieren von Teilen der ionisierten Strahlung 7 aufweist. Beispielsweise ist die Bildgebungsmodalität als röntgenbasierte Bildgebungsmodalität, etwa als röntgenbasiertes Angiographiesystem, ausgestaltet und weist eine Röntgenquelle als Strahlungsquelle 4 sowie einen Röntgendetektor als Strahlungsdetektor 3 auf. Ferner ist ein Patient 5 als abzubildendes Objekt dargestellt.

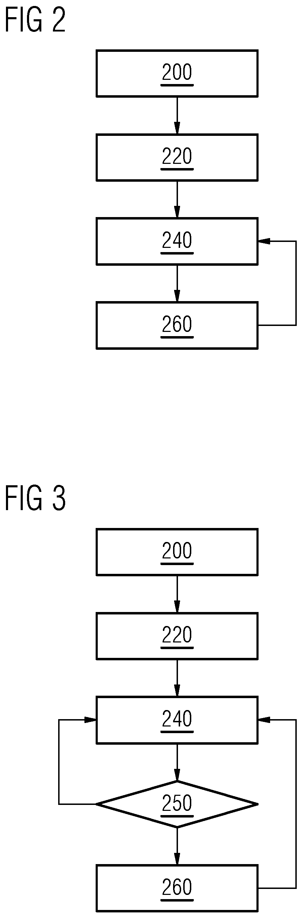

Die wenigstens eine Recheneinheit 2 ist dazu eingerichtet, ein erfindungsgemäßes Verfahren zur Abschätzung einer Strahlenbelastungskenngröße, beispielsweise einer Kerma oder eines DAP, für das Bildgebungssystem 1 durchzuführen. Ein schematisches Ablaufdiagramm einer beispielhaften Ausführungsform eines solchen Verfahrens ist in FIG 2 dargestellt, wobei der Schritt 260 optional ist.

Die Kerma, insbesondere Luft-Kerma, entspricht der pro Masseneinheit, insbesondere in Luft, freigesetzte Energie und wird in Gy gemessen. Sie kann als Produkt der Energie, der Photonenflussrate und der Energieabsorption der Strahlung 7 in Luft bestimmt werden. In homogenen Medien wie Luft ist die Kerma näherungsweise gleich der Dosis. Das DAP, gemessen in Gym² ist definiert als die Kerma multipliziert mit der entsprechenden Fläche, die der Bestrahlung ausgesetzt ist. Kerma und DAP hängen, neben dem Medium, mit dem die Strahlung 7 wechselwirkt, also insbesondere Luft, im Wesentlichen vom Energiespektrum der Strahlung ab. Beide Größen vermitteln die gleiche Information. Wenn die Kerma oder das DAP bekannt ist, kann die jeweils andere Größe daraus abgeleitet werden.

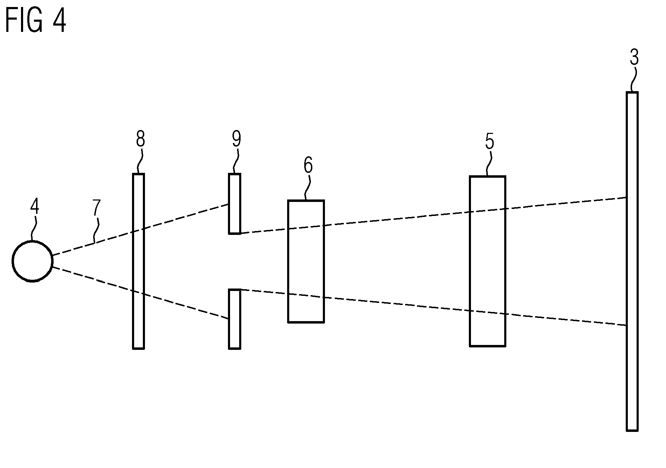

In FIG 4 ist schematisch der Strahlengang von der Strahlungsquelle 4 bis zu dem Strahlungsdetektor 3 für eine weitere beispielhafte Ausführungsform des Bildgebungssystems 1 dargestellt. Dabei sind direkt nach der Strahlungsquelle 4 beispielsweise eine Strahlungsfiltervorrichtung 8, im Falle einer Röntgenröhre beispielsweise ein Filter zur Strahlaufhärtung und/oder ein Keilfilter, auch als Wedge-Filter bezeichnet, und/oder eine Strahlformungsvorrichtung 9, beispielsweise ein Kollimator, angeordnet. Zwischen dem Patienten 5 einerseits und der Strahlungsquelle 4, der Strahlungsfiltervorrichtung 8 und der Strahlformungsvorrichtung 9 anderseits kann eine Messvorrichtung 6, beispielsweise eine DAP-Kammer, zur Messung der Strahlenbelastungskenngröße eingebracht werden.

Im Schritt 200 wird ein Basismodell zur Bestimmung der Strahlenbelastungskenngröße abhängig von einem Satz von Eingangsgrößen erhalten, wobei die Eingangsgrößen wenigstens einen Strahlungsquellenbetriebsparameter beinhalten. In Schritt 220 werden für das Bildgebungssystem 1 gemessene Messdaten, insbesondere mittels der Messvorrichtung 6 gemessene Messdaten, erhalten, welche die Strahlenbelastungskenngröße für eine Vielzahl von verschiedenen Werten des wenigstens einen Strahlungsquellenbetriebsparameters beinhalten. Ein verfeinertes Modell wird erzeugt, indem das Basismodell abhängig von den Messdaten angepasst wird.

In Schritt 240 werden aktuelle Werte für den Satz von Eingangsgrößen für das Bildgebungssystem 1 bestimmt und mittels des verfeinerten Modells wird abhängig von den aktuellen Werten für den Satz von Eingangsgrößen ein Schätzwert die Strahlenbelastungskenngröße bestimmt.

Im optionalen Schritt 260 werden in manchen Ausführungsformen weitere Messdaten, gemessen für das Bildgebungssystem 1, erhalten, welche die Strahlenbelastungskenngröße für eine weitere Vielzahl von verschiedenen Werten des wenigstens einen Strahlungsquellenbetriebsparameters beinhalten. Es wird ein aktualisiertes Modell erzeugt, indem das verfeinerte Modell abhängig von den weiteren Messdaten angepasst wird. Weitere Werte für den Satz von Eingangsgrößen für das Bildgebungssystem 1 werden bestimmt und mittels des aktualisierten Modells wird abhängig von den weiteren Werten für den Satz von Eingangsgrößen ein weiterer Schätzwert die Strahlenbelastungskenngröße bestimmt.

Da die Strahlenbelastungskenngröße, insbesondere das DAP oder die Kerma, von dem Energiespektrum der Strahlung 7 abhängt, kann sie prinzipiell mit Hilfe von Informationen betreffend die Strahlungsquelle 4, insbesondere die Strahlungsquellenbetriebsparameter wie, im Falle einer Röntgenröhre, Spitzenkilovoltspannung, Röhrenstrom, Brennfleckgröße und so weiter, und gegebenenfalls Informationen betreffend die Strahlungsfiltervorrichtung 8, wie Filtermaterialdicke, Keilfilterposition, und so weiter, beziehungsweise betreffend die Strahlformungsvorrichtung 9, etwa eine Ausdehnung des kollimierten Feldes, und Informationen betreffend die Belichtung, wie Belichtungsdauer, Pulsweite und so weiter, geschätzt werden, da diese Information in der Regel bei jeder Aufnahme verfügbar sind. Das Energiespektrum der Strahlungsquelle 4 kann sich jedoch im Laufe der Zeit ändern, beispielsweise durch Alterungseffekte, ebenso wie die Eigenschaften der Strahlungsfiltervorrichtung 8 und der Strahlformungsvorrichtung 9. Durch das verfeinerte Modell werden solche Änderungen implizit berücksichtigt und die Genauigkeit des verfeinerten Models wird gegenüber dem Basismodell daher verbessert.

Das Basismodell selbst kann auf unterschiedliche Weise bereitgestellt werden. Beispielsweise können, etwa während einer Entwicklungsphase, für wenigstens ein Referenzbildgebungssystem gemessene Referenzmessdaten erhalten werden, welche die Strahlenbelastungskenngröße für eine Vielzahl von verschiedenen Werten des wenigstens einen Strahlungsquellenbetriebsparameters beinhalten und das Basismodell basierend auf den Referenzmessdaten erzeugt werden. Hierbei können die Referenzmessdaten sehr umfangreich sein. Beispielsweise kann ein physikalisches Initialmodell zur Bestimmung der Strahlenbelastungskenngröße abhängig von dem Satz von Eingangsgrößen abhängig von den Referenzmess-daten angepasst werden, um das Basismodell zu erzeugen. Das Basismodell kann auch erzeugt werden, indem ein durch maschinelles Lernen trainierbarer Algorithmus basierend auf den Referenzmessdaten trainiert wird. Das Basismodell kann auch von Zeit zu Zeit neu erstellt werden, um Änderungen im System zu berücksichtigen.

Die Messdaten zur Erzeugung des verfeinerten Modells können dann beispielsweise während der Produktion des konkreten Bildgebungssystems 1, insbesondere der Strahlungsquelle 4, erfasst werden, wobei die Messdaten weniger umfangreich sein können als die Referenzmessdaten.

Das Basismodell kann beispielsweise ein physikalisches Modellsein, welches ein ideales Energiespektrum berechnet und daraus die Strahlenbelastungskenngröße ableitet. Das Basismodell kann auch eine Approximation eines solchen physikalischen Modells sein, die beispielsweise durch Polynom- oder Spline-Approximation erhalten wird. Das Basismodell kann auch ein basierend auf maschinellem Lernen trainiertes Modell sein.

Das verfeinerte Modell kann beispielsweise ein basierend auf maschinellem Lernen trainiertes Modell zur Abschätzung der effektiven Parameter für gealterte Komponenten des Bildgebungssystems 1 sein. Zum Beispiel können im verfeinerten Modell solche effektiven Parameter im Vergleich zum Basismodell geändert sein. Im Falle eines Röntgenbildgebungssystem kann beispielsweise ein gealterter Metallfilter aus einem ersten Material, etwa Kupfer, durch einen effektiven Metallfilter aus einem anderen Material und/oder mit einer anderen Materialdicke angenähert werden. Das verfeinerte Modell kann auch dadurch implementiert werden, dass es das Basismodell enthält und die Ausgabe des Basismodells korrigiert, zum Beispiel durch Multiplikation mit skalaren Korrekturfaktoren.

In FIG 3 ist ein schematisches Ablaufdiagramm einer weiteren beispielhaften Ausführungsform eines erfindungsgemäßen Verfahrens dargestellt, wobei die Schritte 200, 220, 240 und 260 denen des Verfahrens aus FIG 2 entspricht.

Im Verfahren gemäß FIG 3 wird nach Schritt 240 und vor Schritt 260 eine Konsistenzprüfung durchgeführt. Der Schritt 260 wird nur dann beziehungsweise erst dann durchgeführt, wenn abhängig von einem Ergebnis der Konsistenzprüfung festgestellt wird, dass eine Inkonsistenz vorliegt, anderenfalls wird das verfeinerte Modell weiter benutzt.

Um die Konsistenzprüfung durchzuführen, werden mittels des Bildgebungssystems 1 erzeugte Bilddaten, welche einen Patienten 5 darstellen, erhalten und es wird ein Körpermodell für den Körper des Patienten 5 erhalten. Die Konsistenzprüfung wird als Kreuzvalidierung der den Bilddaten, des Körpermodells, den für die Erzeugung der Bilddaten verwendeten Werten für den Satz von Eingangsgrößen, und des verfeinerten Modells durchgeführt.

Beispielsweise können abhängig von dem Körpermodell, abhängig von den für die Erzeugung der Bilddaten verwendeten Werten für den Satz von Eingangsgrößen und abhängig von dem verfeinerten Modell simulierte Bilddaten erzeugt werden und zur Konsistenzprüfung die simulierten Bilddaten mit den Bilddaten abgeglichen werden. Zur Erzeugung der simulierten Bilddaten können beispielsweise Renderverfahren und/oder physikalische Simulationen eingesetzt werden. Weichen die simulierten Bilddaten gemäß vorgegebenen Grenzen zu stark von den Bilddaten ab, so kann auf das Vorliegen einer Inkonsistenz geschlossen werden und dementsprechend die Aktualisierung des verfeinerten Modells durchgeführt werden.

Zusätzlich oder alternativ kann auch abhängig von den Bilddaten, abhängig von dem Körpermodell und abhängig von den für die Erzeugung der Bilddaten verwendeten Werten für den Satz von Eingangsgrößen ein Vergleichsmodell erzeugt werden und zur Konsistenzprüfung das Vergleichsmodell mit dem verfeinerten Modell abgeglichen werden. Das Vergleichsmodell kann beispielsweise durch inverse Renderverfahren und/oder physikalische Simulationen ermittelt werden. Weicht das Vergleichsmodell gemäß vorgegebenen Grenzen zu stark von dem verfeinerten Modell ab, so kann auf das Vorliegen einer Inkonsistenz geschlossen werden und dementsprechend die Aktualisierung des verfeinerten Modells durchgeführt werden.

Weiterhin zusätzlich oder alternativ kann abhängig von den Bilddaten, abhängig von den für die Erzeugung der Bilddaten verwendeten Werten für den Satz von Eingangsgrößen und abhängig von dem verfeinerten Modell ein Vergleichskörper-modell erzeugt werden und zur Konsistenzprüfung mit dem Körpermodell abgeglichen werden. Das Vergleichskörpermodell kann beispielsweise durch inverse Renderverfahren und/oder physikalische Simulationen ermittelt werden. Weicht das Vergleichskörpermodell gemäß vorgegebenen Grenzen zu stark von dem Körpermodell ab, so kann auf das Vorliegen einer Inkonsistenz geschlossen werden und dementsprechend die Aktualisierung des verfeinerten Modells durchgeführt werden.

Durch die Konsistenzprüfung können Unstimmigkeiten oder Abweichungen zwischen der jeweiligen Vorhersage und tatsächlichen Eingabedaten aufgedeckt werden. Durch inverses Rendern, auch als differenzierbares Rendern bezeichnet, und/oder physikalische Simulationen kann die Konsistenzprüfung auch als Lösung eines Optimierungsproblems erfolgen. Es ist auch möglich, den jeweiligen Abgleich der Daten durch einen entsprechend trainierten Algorithmus durchzuführen, der beispielsweise als Eingabedaten die Bilddaten und die simulierten Bilddaten erhält und als Ausgabe einen Konsistenzwert prädiziert. Analog können als Eingabedaten das Vergleichsmodell und das verfeinerte Modell beziehungsweise das Vergleichskörpermodell und das Körpermodell verwendet werden.

Durch die Durchführung der Kreuzvalidierung ist es außerdem möglich, die Störfaktoren in der gesamten Verfahrenskette zu identifizieren, um sicherzustellen, dass die richtige Aktion vorgeschlagen wird. Wenn zum Beispiel das Körpermodell der wichtigste Grund für eine Inkonsistenz ist, ist gegebenenfalls eine Aktualisierung des verfeinerten Modells nicht erforderlich.

Alternativ zu der beschriebenen Kreuzvalidierung kann ein entsprechend durch maschinelles Lernen trainierter Algorithmus eingesetzt werden, um die Notwendigkeit der Aktualisierung des verfeinerten Modells zu prädizieren. Eine mögliche Implementierung könnte ein Algorithmus sein, das als Eingaben Parameter zur Definition des verfeinerten Modells, Parameter zur Definition des Körpermodells, Parameter zur Definition der Systemgeometrie, sowie die für die Erzeugung der Bilddaten verwendeten Werte und als Ausgabe einen Konsistenzwert vorhersagt.

Durch die Möglichkeit, die Notwendigkeit zur Aktualisierung des verfeinerten Modells flexibel und abhängig von den aktuellen Umständen zu bestimmen, können unnötige Maßnahmen und daher Zeitaufwand und Kosten gespart werden. Ferner kann der Aktualisierungsbedarf schnell erkannt werden und darauf angemessen reagiert werden.

Unabhängig vom grammatikalischen Geschlecht eines bestimmten Begriffes sind Personen mit männlicher, weiblicher oder anderer Geschlechteridentität mit umfasst. Dies betrifft insbesondere auch den Begriff "Patient".

## Patentansprüche

1. Verfahren zur Abschätzung einer Strahlenbelastungskenngrö-ße für ein Bildgebungssystem (1) mit einer Strahlungsquelle (4), wobei
- ein Basismodell zur Bestimmung der Strahlenbelastungskenngröße abhängig von einem Satz von Eingangsgrößen, welcher wenigstens einen Strahlungsquellenbetriebsparameter beinhaltet, erhalten wird;
- für das Bildgebungssystem (1) gemessene Messdaten erhalten werden, welche die Strahlenbelastungskenngröße für eine Vielzahl von verschiedenen Werten des wenigstens einen Strahlungsquellenbetriebsparameters beinhalten;
- ein verfeinertes Modell erzeugt wird, indem das Basismodell abhängig von den Messdaten angepasst wird;
- aktuelle Werte für den Satz von Eingangsgrößen für das Bildgebungssystem (1) bestimmt werden;
- mittels des verfeinerten Modells abhängig von den aktuellen Werten für den Satz von Eingangsgrößen ein Schätzwert für die Strahlenbelastungskenngröße bestimmt wird;
**dadurch gekennzeichnet, dass**
nach Ablauf eines Aktualisierungszeitraums nach einer Messung der Messdaten
- weitere Messdaten, gemessen für das Bildgebungssystem (1), erhalten werden, welche die Strahlenbelastungskenngröße für eine weitere Vielzahl von verschiedenen Werten des wenigstens einen Strahlungsquellenbetriebsparameters beinhalten;
- ein aktualisiertes Modell erzeugt wird, indem das verfeinerte Modell abhängig von den weiteren Messdaten angepasst wird;
- weitere Werte für den Satz von Eingangsgrößen für das Bildgebungssystem (1) bestimmt werden; und
- mittels des aktualisierten Modells abhängig von den weiteren Werten für den Satz von Eingangsgrößen ein weiterer Schätzwert für die Strahlenbelastungskenngröße bestimmt wird;
- mittels des Bildgebungssystems (1) erzeugte Bilddaten, welche ein abzubildendes Objekt (5) darstellen, erhalten werden;
- ein Körpermodell für das Objekt (5) erhalten wird;
- abhängig von den Bilddaten, abhängig von dem Körpermodell, abhängig von für die Erzeugung der Bilddaten verwendeten Werten für den Satz von Eingangsgrößen, und abhängig von dem verfeinerten Modell, eine Konsistenzprüfung durchgeführt wird; und
- abhängig von einem Ergebnis der Konsistenzprüfung festgestellt wird, ob der Aktualisierungszeitraum abgelaufen ist.

2. Verfahren nach Anspruch 1, wobei
- für wenigstens ein Referenzbildgebungssystem gemessene Referenzmessdaten erhalten werden, welche die Strahlenbelastungskenngröße für eine Vielzahl von verschiedenen Werten des wenigstens einen Strahlungsquellenbetriebsparameters beinhalten; und
- das Basismodell basierend auf den Referenzmessdaten erzeugt wird.

3. Verfahren nach Anspruch 2, wobei
- ein physikalisches Initialmodell zur Bestimmung der Strahlenbelastungskenngröße abhängig von dem Satz von Eingangsgrößen erhalten wird; und
- das Basismodell erzeugt wird, indem das physikalische Initialmodell abhängig von den Referenzmessdaten angepasst wird.

4. Verfahren nach Anspruch 2, wobei das Basismodell erzeugt wird, indem ein durch maschinelles Lernen trainierbarer Algorithmus basierend auf den Referenzmessdaten trainiert wird.

5. Verfahren nach einem der vorhergehenden Ansprüche, wobei
- der Satz von Eingangsgrößen wenigstens einen Zustands- und/oder Materialparameter für eine Strahlungsfiltervorrichtung (8) und/oder eine Strahlformungsvorrichtung (9) beinhaltet; und
- die Messdaten die Strahlenbelastungskenngröße für eine Vielzahl von verschiedenen Werten des wenigstens einen Zustands- und/oder Materialparameters beinhalten.

6. Verfahren nach einem der vorhergehenden Ansprüche, wobei
- der Satz von Eingangsgrößen wenigstens einen Belichtungsparameter beinhaltet; und
- die Messdaten die Strahlenbelastungskenngröße für eine Vielzahl von verschiedenen Werten des wenigstens einen Belichtungsparameter beinhalten.

7. Verfahren nach einem der vorhergehenden Ansprüche, wobei die Strahlenbelastungskenngröße ein Dosisflächenprodukt oder eine Kerma oder eine von dem Dosisflächenprodukt und/oder der Kerma abhängige Größe ist.

8. Verfahren nach einem der vorhergehenden Ansprüche, wobei
- abhängig von dem Körpermodell, abhängig von den für die Erzeugung der Bilddaten verwendeten Werten für den Satz von Eingangsgrößen und abhängig von dem verfeinerten Modell simulierte Bilddaten erzeugt werden und zur Konsistenzprüfung die simulierten Bilddaten mit den Bilddaten abgeglichen werden; und/oder
- abhängig von den Bilddaten, abhängig von dem Körpermodell und abhängig von den für die Erzeugung der Bilddaten verwendeten Werten für den Satz von Eingangsgrößen ein Vergleichsmodell erzeugt wird und zur Konsistenzprüfung das Vergleichsmodell mit dem verfeinerten Modell abgeglichen wird; und/oder
- abhängig von den Bilddaten, abhängig von den für die Erzeugung der Bilddaten verwendeten Werten für den Satz von Eingangsgrößen und abhängig von dem verfeinerten Modell ein Vergleichskörpermodell erzeugt wird und zur Konsistenzprüfung das Vergleichskörpermodell mit dem Körpermodell abgeglichen wird.

9. Verfahren nach einem der vorhergehenden Ansprüche, wobei die Konsistenzprüfung eine Anwendung eines durch maschinelles Lernen trainierten weiteren Algorithmus auf von den Bilddaten, dem Körpermodell, den für die Erzeugung der Bilddaten verwendeten Werten für den Satz von Eingangsgrößen, und dem verfeinerten Modell abhängige Eingabedaten beinhaltet.

10. Verfahren nach einem der vorhergehenden Ansprüche, wobei das Bildgebungssystem (1) ein röntgenbasiertes Bildgebungssystem (1) ist und die Strahlungsquelle (4) eine Röntgenstrahlungsquelle (4) ist.

11. Datenverarbeitungsvorrichtung (2) mit wenigstens einer Recheneinheit, die dazu eingerichtet ist, ein Verfahren nach einem der vorhergehenden Ansprüche durchzuführen.

12. Bildgebungssystem (1) aufweisend eine Strahlungsquelle (4) zum Erzeugen ionisierender Strahlung (7), einen Strahlungsdetektor (3) zum Detektieren von Teilen der ionisierten Strahlung (7), sowie eine Datenverarbeitungsvorrichtung (2) nach Anspruch 11.

13. Computerprogrammprodukt mit Befehlen, die bei Ausführung durch eine Datenverarbeitungsvorrichtung (2) die Datenverarbeitungsvorrichtung (2) dazu veranlassen, ein Verfahren nach einem der Ansprüche 1 bis 10 durchzuführen.

## Claims

1. Method for estimating a radiation load quantity for an imaging system (1) having a radiation source (4), wherein
- a base model is obtained for determining the radiation load quantity depending on a set of input variables, which include at least one radiation-source operating parameter;
- measurement data measured for the imaging system (1) is obtained which includes the radiation load quantity for a multiplicity of different values of the at least one radiation-source operating parameter;
- a refined model is generated by adapting the base model depending on the measurement data;
- actual values of the set of input variables are defined for the imaging system (1);
- the refined model is used to determine, depending on the actual values of the set of input variables, an estimated value of the radiation load quantity;
**characterised in that**
after an update period has elapsed after a measurement of the measurement data,
- further measurement data measured for the imaging system (1) is obtained which includes the radiation load quantity for a further multiplicity of different values of the at least one radiation-source operating parameter;
- an updated model is generated by adapting the refined model depending on the further measurement data;
- further values of the set of input variables are defined for the imaging system (1); and
- the updated model is used to determine, depending on the further values of the set of input variables, a further estimated value of the radiation load quantity;
- image data generated by means of the imaging system (1) and that represents an object (5) to be imaged is obtained;
- a body model for the object (5) is obtained;
- a consistency check is carried out on the basis of the image data, on the basis of the body model, on the basis of values of the set of input variables, which values were used to generate the image data, and on the basis of the refined model; and
- depending on the result of the consistency check, it is ascertained whether the update period has elapsed.

2. Method according to claim 1, wherein
- reference measurement data measured for at least one reference imaging system is obtained which includes the radiation load quantity for a multiplicity of different values of the at least one radiation-source operating parameter; and
- the base model is generated on the basis of the reference measurement data.

3. Method according to claim 2, wherein
- a physical initial model is obtained for determining the radiation load quantity depending on the set of input variables; and
- the base model is generated by adapting the physical initial model depending on the reference measurement data.

4. Method according to claim 2, wherein the base model is generated by using the reference measurement data as the basis for training an algorithm trainable by machine learning.

5. Method according to one of the preceding claims, wherein
- the set of input variables includes at least one state parameter and/or material parameter for a radiation filtering apparatus (8) and/or a beam-forming apparatus (9); and
- the measurement data includes the radiation load quantity for a multiplicity of different values of the at least one state parameter and/or material parameter.

6. Method according to one of the preceding claims, wherein
- the set of input variables includes at least one exposure parameter; and
- the measurement data includes the radiation load quantity for a multiplicity of different values of the at least one exposure parameter.

7. Method according to one of the preceding claims, wherein the radiation load quantity is a dose area product or a kerma or a variable that depends on the dose area product and/or the kerma.

8. Method according to one of the preceding claims, wherein
- simulated image data is generated on the basis of the body model, on the basis of the values of the set of input variables, which values were used to generate the image data, and on the basis of the refined model, and the simulated image data is compared with the image data for the purpose of the consistency check; and/or
- a comparative model is generated on the basis of the image data, on the basis of the body model, and on the basis of the values of the set of input variables, which values were used to generate the image data, and the comparative model is compared with the refined model for the purpose of the consistency check; and/or
- a comparative body model is generated on the basis of the image data, on the basis of the values of the set of input variables, which values were used to generate the image data, and on the basis of the refined model, and the comparative body model is compared with the body model for the purpose of the consistency check.

9. Method according to one of the preceding claims, wherein the consistency check includes applying a further algorithm, which is trained by machine learning, to input data that depends on the image data, the body model, the values of the set of input variables, which values were used to generate the image data, and the refined model.

10. Method according to one of the preceding claims, wherein the imaging system (1) is an X-ray based imaging system (1), and the radiation source (4) is an X-ray radiation source (4).

11. Data processing apparatus (2) having at least one computing unit, which is configured to perform a method according to one of the preceding claims.

12. Imaging system (1) having a radiation source (4) for generating ionising radiation (7), a radiation detector (3) for detecting portions of the ionising radiation (7), and a data processing apparatus (2) as claimed in claim 11.

13. Computer program product comprising commands which, on execution by a data processing apparatus (2), cause the data processing apparatus (2) to perform a method according to one of claims 1 to 10.

## Revendications

1. Procédé d'estimation d'une grandeur caractéristique de la charge de faisceau pour un système (1) d'imagerie ayant une source (4) de rayonnement, dans lequel
- on obtient un modèle de base pour la détermination de la grandeur caractéristique de charge de faisceau en fonction d'un ensemble de grandeurs d'entrée, qui contient au moins un paramètre de fonctionnement de source de rayonnement ;
- on obtient des données de mesure, mesurées pour le système (1) d'imagerie, qui contiennent la grandeur caractéristique de charge de faisceau pour une pluralité de valeurs différentes du au moins un paramètre de fonctionnement de source de rayonnement ;
- on produit un modèle raffiné en adaptant le modèle de base en fonction des données de mesure ;
- on détermine des valeurs en cours de l'ensemble de grandeurs d'entrée pour le système (1) d'imagerie ;
- au moyen du modèle raffiné on détermine, en fonction des valeurs en cours pour le jeu de grandeurs d'entrée, une valeur d'estimation de la grandeur caractéristique de charge de faisceau ;
**caractérisé en ce que**,
après l'expiration d'un laps de temps de mise à jour après une mesure des données de mesure,
- on obtient d'autres données de mesure, mesurées pour le système (1) d'imagerie, qui contiennent la grandeur caractéristique de charge de faisceau pour une autre pluralité de valeurs différentes du au moins un paramètre de fonctionnement de source de rayonnement ;
- on produit un modèle mis à jour, en adaptant le modèle raffiné en fonction des autres données de mesure ;
- on détermine d'autres valeurs pour l'ensemble de grandeurs d'entrée pour le système (1) d'imagerie ; et
- au moyen du modèle mis à jour, on détermine, en fonction des autres valeurs pour l'ensemble de grandeurs d'entrée, une autre valeur d'estimation pour la grandeur caractéristique de charge de faisceau ;
- on obtient des données d'image produites au moyen du système (1) d'imagerie, qui représente un objet (5) à représenter ;
- on obtient un modèle de corps pour l'objet (5) ;
- on effectue un contrôle de cohérence, en fonction des données d'image, en fonction du modèle de corps, en fonction de valeurs, utilisées pour la production des données d'image, pour l'ensemble de grandeurs d'entrée et en fonction du modèle raffiné ; et
- on détermine, en fonction d'un résultat du contrôle de cohérence, si le laps de temps de mise à jour est expiré.

2. Procédé suivant la revendication 1, dans lequel
- on obtient des données de mesure de référence, mesurées pour au moins un système d'imagerie de référence, qui contiennent la grandeur caractéristique de charge de faisceau pour une pluralité de valeurs différentes du au moins un paramètre de fonctionnement de source de rayonnement ; et
- on produit le modèle de base sur la base des données de mesure de référence.

3. Procédé suivant la revendication 2, dans lequel
- on obtient un modèle physique initial de détermination de la grandeur caractéristique de charge de faisceau en fonction de l'ensemble de grandeurs d'entrée ; et
- on produit le modèle de base, en adaptant le modèle physique initial en fonction des données de mesure de référence.

4. Procédé suivant la revendication 2, dans lequel on produit le modèle de base en entraînant sur la base des données de mesure de référence un algorithme pouvant être entraîné par apprentissage automatique.

5. Procédé suivant l'une des revendications précédentes, dans lequel
- l'ensemble de grandeurs d'entrée contient au moins un paramètre d'état et/ou de matériau d'un dispositif (8) de filtrage de rayonnement et/ou un dispositif (9) de formation de faisceau ; et
- les données de mesure contiennent la grandeur caractéristique de charge de faisceau pour une pluralité de valeurs différentes du au moins un paramètre d'état et/ou de matériau.

6. Procédé suivant l'une des revendications précédentes, dans lequel
- l'ensemble de grandeurs d'entrée contient au moins un paramètre d'exposition ; et
- les données de mesure contiennent la grandeur caractéristique de charge de faisceau pour une pluralité de valeurs différentes du au moins un paramètre d'exposition.

7. Procédé suivant l'une des revendications précédentes, dans lequel la grandeur caractéristique de charge de faisceau est un produit dose surface ou un kerma ou une grandeur, qui dépend du produit dose surface et/ou du kerma.

8. Procédé suivant l'une des revendications précédentes, dans lequel
- en fonction du modèle de corps, en fonction des valeurs utilisées pour la production des données d'image pour le jeu de grandeurs d'entrée et en fonction du modèle raffiné, on produit des données d'image simulées et pour le contrôle de cohérence, on ajuste les données d'image simulées aux données d'image ; et/ou
- en fonction des données d'image, en fonction du modèle de corps et en fonction des valeurs, utilisées pour la production des données d'image, pour l'ensemble de grandeurs d'entrée, on produit un modèle de comparaison et pour le contrôle de cohérence on ajuste le modèle de comparaison au modèle raffiné ; et/ou
- en fonction des données d'image, en fonction des valeurs utilisées pour la production des données d'image pour l'ensemble de grandeurs d'entrée et en fonction du modèle raffiné, on produit un modèle de corps de comparaison et, pour le contrôle de cohérence, on ajuste le modèle de corps de comparaison au modèle de corps.

9. Procédé suivant l'une des revendications précédentes, dans lequel le contrôle de cohérence contient une application d'un autre algorithme entraîné par l'apprentissage automatique à des valeurs, utilisées par les données d'image, le modèle de corps, des valeurs utilisées pour la production de données d'image, pour l'ensemble de grandeurs d'entrée, et des données d'entrée en fonction du modèle raffiné.

10. Procédé suivant l'une des revendications précédentes, dans lequel le système (1) d'imagerie est un système (1) d'imagerie à base de rayons X et la source (4) de rayonnement est une source (4) de rayonnement à rayons X.

11. Dispositif (2) de traitement de données comprenant au moins une unité informatique, qui est agencée pour exécuter un procédé suivant l'une des revendications précédentes.

12. Système (1) d'imagerie comportant une source (4) de rayonnement pour la production de rayonnement (7) ionisé, un détecteur (3) de rayonnement pour la détection de particules du rayonnement (7) ionisé ainsi qu'un dispositif (2) de traitement de données suivant la revendication 11.

13. Produit de programme d'ordinateur, comprenant des instructions, qui, lors de l'exécution par un dispositif (2) de traitement de données, font que le dispositif (2) de traitement de données exécute un procédé suivant l'une des revendications 1 à 10.
